# EUROPÄISCHE PATENTSCHRIFT

(11) **EP 3 573 951 B1**
(45) Veröffentlichungstag und Bekanntmachung des Hinweises auf die Patenterteilung: **28.10.2020**
(21) Anmeldenummer: 17835853.7
(22) Anmeldetag: 22.12.2017
(51) Int. Cl.: C07C 323/52

(54) **VERFAHREN ZUR HERSTELLUNG VON DIHYDROLIPONSÄURE**
PROCESS FOR THE PREPARAITON OF DIHYDROLIPOIC ACID
PROCÉDÉ POUR LA PRÉPARATION DE L'ACIDE DIHYDROLIPOIQUE

(30) Priorität: 28.01.2017 DE 102017000811
(43) Veröffentlichungstag der Anmeldung: 04.12.2019
(73) Patentinhaber: AlzChem Trostberg GmbH, 83308 Trostberg (DE)
(72) Erfinder: SCHNAPPERELLE, Ingo, 83512 Wasserburg (DE); WINKLER, Stephan, 83352 Altenmarkt (DE); SANS, Jürgen, 83308 Trostberg (DE); THALHAMMER, Franz, 83308 Trostberg (DE)
(74) Vertreter: Weickmann & Weickmann PartmbB
(86) Internationale Anmeldenummer: PCT/EP2017/084511
(87) Internationale Veröffentlichungsnummer: WO 2018/137874

(56) Entgegenhaltungen:
- EP-A1- 0 763 533
- WO-A2-02/44163
- S-J ZHANG ET. AL.: "Synthesis and anticancer evaluation of alpha-lipoic acid derivatives.", BIOORGANIC AND MEDICINAL CHEMISTRY LETTERS, Bd. 20, 3. April 2010 (2010-04-03), Seiten 3078-3083, XP002778647,

## Beschreibung

Die vorliegende Erfindung betrifft ein mehrstufiges Verfahren zur Herstellung von Dihydroliponsäure, das insbesondere in Form eines Eintopfverfahrens und ohne Isolierung von Zwischenprodukten durchgeführt werden kann.

Dihydroliponsäure (C₈H₁₆O₂S₂; 6,8-Disulfanyloctansäure; CAS-Nr. 462-20-4) 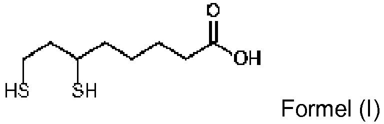 ist eine schwefelhaltige Carbonsäure, die als offenkettige Form der alpha-Liponsäure (C₈H₁₄O₂S₂; 5-(1,2-Dithiolan-3-yl)-pentansäure; CAS-Nr. 1077-28-7) bezeichnet werden kann und die im Vergleich zur alpha-Liponsäure anstelle einer S-S-Schwefelbrücke zwei SH-Gruppen aufweist. Sowohl die Dihydroliponsäure als auch die alpha-Liponsäure kann in zwei enantiomeren Formen vorliegen (vgl. R-Dihydroliponsäure (Formel Ia) und S-Dihydroliponsäure (Formel Ib).

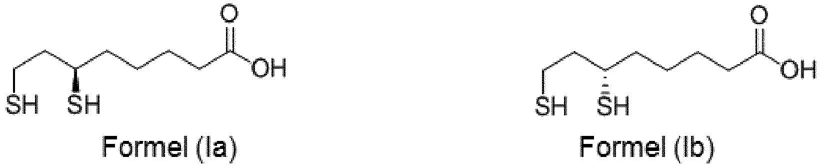

Dihydroliponsäure ist eine im menschlichen Organismus vorkommende Verbindung, die mit alpha-Liponsäure, einer seit geraumer Zeit auch als Wachstumsfaktor in Mikroorganismen bekannten Substanz, die zudem in geringen Konzentrationen auch in höheren Pflanzen und Tieren vorkommt, im physiologischen Gleichgewicht steht. Physiologisch betrachtet ist die Dihydroliponsäure der Redoxpartner der alpha-Liponsäure. Durch die Entdeckung neuer Eigenschaften dieses natürlichen Redoxpaares sind die beiden Naturstoffe wieder verstärkt in das Interesse von Biologie, Biochemie, Medizin, Ernährungswissenschaft und Technik gerückt.

Die chemische Synthese von Dihydroliponsäure ist seit längerer Zeit etabliert und kann prinzipiell auf unterschiedlichen Reaktionswegen realisiert werden. So wird in J. Am. Chem. Soc. 1955, 77, 416-419 ein Verfahren zur Herstellung von Dihydroliponsäure beschrieben, in dem 6,8-Dichloroctansäureester mit Benzylmercaptan unter alkalischen Bedingungen in ethanolischer Lösung umgesetzt wird. Anschließend werden durch eine Reduktion mit Natrium in flüssigem Ammoniak beide Benzylreste abgespalten und nach destillativer Aufreinigung das Produkt in einer Ausbeute von 82% erhalten.

Weiterhin wird mit den internationalen Patentanmeldungen WO 02/10151 A2 und WO 02/44163 A2 ein Verfahren zur Herstellung von Dihydroliponsäure beschrieben, in dem 6,8-Mesyloxyoctansäureester mit einem Gemisch aus Natriumsulfid und Schwefel umgesetzt und anschließen eine Reduktion mittels Natriumborhydrid durchgeführt wird. Das resultierende Reaktionsgemisch wird in einer Vielzahl von Reinigungsschritten aufgearbeitet. Eine Destillation der Dihydroliponsäure ist notwendig, um Polymere und Verunreinigungen abzutrennen.

Auch die Verwendung des Kalium-Salzes der Thioessigsäure ist zur Einführung der SH-Funktionalität beschrieben (Z. Naturforschung B. 1999, 54, 655-661). Hierbei wird 6,8-Mesyloxyoctansäureester in Dimethylformamid mit Kaliumthioacetat umgesetzt und anschließend durch alkalische Hydrolyse Acetyl-Gruppen abgespalten. Dihydroliponsäure wird in einer Ausbeute von 86% erhalten.

Weiterhin wird in US 5,489,694 A zur Synthese von Dihydroliponsäure und alpha-Liponsäure Cyclohexanon in Gegenwart eines Radikalstarters mit einem Vinylalkylether umgesetzt, das resultierende 2-Alkoxyethylcyclohexanon anschließend mit einer Persäure in einer Baeyer-Villiger-Reaktion zu einem Lacton umgesetzt, welches im Anschluss mit Thioharnstoff und in Gegenwart von Bromwasserstoffsäure oder lodwasserstoffsäure zu Dihydroliponsäure reagiert. Die so erhaltene rohe Dihydroliponsäure wird entweder isoliert oder in Gegenwart eines Eisen-(III)-Katalysators zu alpha-Liponsäure oxidiert. Die rohe alpha-Liponsäure wird schließlich in einem Dünnschichtverdampfer durch kontinuierliche Destillation bei 0,2 bis 0,02 mbar und 60 bis 200 °C destilliert, und das Destillat kristallisiert (bevorzugt aus Diisopropylether oder einem Gemisch aus Hexan oder Ethylacetat). Die Nachteile dieser synthetischen Route sind die geringe Selektivität der radikalischen Addition von Cyclohexanon an das Vinylderivat, das Auftreten von Produktgemischen nach der Baeyer-Villiger-Oxidation, und wiederum die Notwendigkeit einer destillativen Reinigung der thermisch labilen Produkte.

EP 0 763 533 A1 beschreibt die Herstellung und Verwendung der reinen Enantiomere der 8-Chlor-6-Sulfonyloxy-Oktansäure und ihrer Alkylester und der reinen Enantiomere der 6,8-Dichlor-Oktansäure und ihrer Alkylester als Zwischenprodukte bei der Synthese der Enantiomere der alpha-Liponsäure sowie ein Verfahren zur Überführung beider Enantiomere der 8-Chlor-6-Hydroxy-Oktansäure in ein Enantiomer der alpha-Liponsäure.

Der vorliegenden Erfindung liegt die Aufgabe zugrunde, ein neuartiges Verfahren zur Herstellung von Dihydroliponsäure bereitzustellen, das in einfacher Art und Weise auch großtechnisch angewendet werden kann, die Reaktion von Nebenprodukten weitgehend zurückdrängt sowie eine hohe Raum-Zeit-Ausbeute ermöglicht.

Gelöst werden diese Aufgaben durch ein Verfahren gemäß Anspruch 1. Bevorzugte Ausführungsformen der Erfindung sind in den Unteransprüchen angegeben, die wahlweise miteinander kombiniert werden können.

Somit ist gemäß einer ersten Ausführung ein mehrstufiges Verfahren zur Herstellung von Dihydroliponsäure Gegenstand der vorliegenden Erfindung, in dem
a) in einer ersten Verfahrensstufe ein Ester der 6,8-Dichloroctansäure gemäß allgemeiner Formel (II) mit einem Gemisch umfassend Natriumsulfid und Schwefel zur Reaktion gebracht wird, wobei für Formel (II) gilt 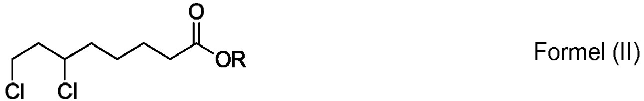 wobei für den Rest R gilt:
   R = C1- bis C6-Alkyl,
   und wobei die Reaktion in einem alkoholischen Lösungsmittelgemisch bei einer Temperatur im Bereich von 80 °C bis 130 °C und einem Druck im Bereich von 0,12 MPa bis 0,60 MPa durchgeführt wird,
b) in einer zweiten Verfahrensstufe das aus der ersten Verfahrensstufe erhaltene Reaktionsgemisch einer Reduktion unterworfen wird, und
c) in einer dritten Verfahrensstufe das aus der zweiten Verfahrensstufe resultierende Reaktionsgemisch mittels einer Mineralsäure auf einen pH-Wert im Bereich von pH 1,5 bis < 7 eingestellt und Dihydroliponsäure freigesetzt wird.

Erfindungswesentlich wird das Verfahren derart durchgeführt, dass die erste Verfahrensstufe in einem alkoholischen Lösungsmittelgemisch bei einer Temperatur im Bereich von 80 °C bis 130 °C und bei einem Druck von 0,12 MPa bis 0,60 MPa durchgeführt wird. Bevorzugt wird das Verfahren derart durchgeführt, dass die erste Verfahrensstufe in einem alkoholischen Lösungsmittelgemisch bei einer Temperatur im Bereich von 100 °C bis 130 °C und bei einem Druck von 0,12 MPa bis 0,60 MPa, ganz besonders bevorzugt bei einer Temperatur im Bereich von 110 °C bis 130 °C und bei einem Druck von 0,25 MPa bis 0,50 MPa durchgeführt wird. Soweit nicht anders angegeben handelt es sich bei den hierin angegebenen Druckwerten um absolute Druckwerte.
Überraschender Weise hat sich gezeigt, dass bei derartigen Reaktionsbedingungen der Anteil an polymeren Verbindungen, die als Nebenprodukt der Synthese zwangsläufig resultieren, deutlich gesenkt werden kann. Somit kann durch Anwendung des erfindungsgemäßen Verfahrens eine deutlich höhere Raum-Zeit-Ausbeute in Bezug auf die eingesetzten Edukte realisiert werden. Zudem konnte vollkommen überraschend festgestellt werden, dass bei den eingestellten Reaktionsbedingungen keine zusätzlichen Nebenprodukte entstehen. Die Bildung der polymeren Verbindungen wird somit gänzlich zugunsten des gewünschten Produktes zurück gedrängt. Diese Tatsachen waren in Summe vollkommend überraschend und in keiner Weise vorhersehbar.

Im Zusammenhang mit der vorliegenden Erfindung soll dabei unter C1- bis C6-Alkyl ein linearer oder verzweigter Alkylrest mit einer Anzahl von bis zu 6 Kohlenstoffatomen verstanden sein, der insbesondere die allgemeinen Formel CₙH₂ₙ₊₁ aufweist, wobei n = 1 bis 6 bedeuted. Dabei ist bevorzugt vorgesehen, dass C1- bis C6-Alkyl Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl oder 1-Ethylpropyl oder n-Hexyl bedeutet.

Hervorzuheben ist an dieser Stelle, dass das erfindungsgemäße Verfahren sowohl zur Herstellung von racemischer Dihydroliponsäure als auch zur Herstellung von enantiomerenreiner R-Dihydroliponsäure oder enantiomerenreiner S-Dihydroliponsäure verwendet werden kann. Bei der Herstellung dieser Substanzen muss dann der als Edukt für das Verfahren dienende Dichloroctansäurester in der entsprechenden Form verwendet werden. So kann beispielsweise gemäß der vorliegenden Erfindung racemische Dihydroliponsäure aus racemischen Dichloroctansäureestern, R-Dihydroliponsäure aus S-Dichloroctansäureestern sowie S-Dihydroliponsäure aus R-Dichloroctansäureestern hergestellt werden.

Gemäß einer bevorzugten Ausführung der vorliegenden Erfindung ist somit auch ein Verfahren Gegenstand der vorliegenden Erfindung in dem als Ester der Dichloroctansäure gemäß Formel (II) racemische 6,8-Dichloroctansäureethylester, enantiomerenreine R-6,8-Dichloroctansäureethylester, enantiomerenreine S-6,8-Dichloroctansäureethylester oder beliebige Mischungen daraus eingesetzt werden.

Unabhängig hiervon ist somit auch ein mehrstufiges Verfahren zur Herstellung von R-Dihydroliponsäure oder S-Dihydroliponsäure oder einem Gemisch hiervon Gegenstand der vorliegenden Erfindung, in dem
a) in einer ersten Verfahrensstufe ein Ester der 6,8-Dichloroctansäure gemäß allgemeiner Formel (IIa) (S-Dichloroctansäureester) oder der allgemeinen Formel (IIb) (R-Dichloroctansäureester) oder ein Gemisch hiervon mit einem Gemisch umfassend Natriumsulfid und Schwefel zur Reaktion gebracht wird, wobei für die Formeln (IIa) und (IIb) gilt 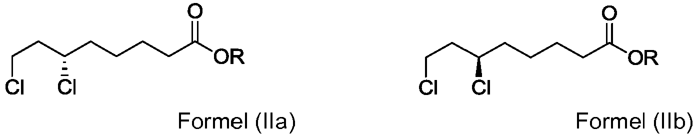 wobei für den Rest R gilt:
   R = C1- bis C6-Alkyl,
   und wobei die Reaktion der ersten Verfahrensstufe in einem alkoholischen Lösungsmittelgemisch bei einer Temperatur im Bereich von 80 °C bis 130 °C und einem Druck im Bereich von 0,12 MPa bis 0,60 MPa durchgeführt wird,
b) in einer zweiten Verfahrensstufe das aus der ersten Verfahrensstufe erhaltene Reaktionsgemisch einer Reduktion unterworfen wird, und
c) in einer dritten Verfahrensstufe das aus der zweiten Verfahrensstufe resultierende Reaktionsgemisch mittels einer Mineralsäure auf einen pH-Wert im Bereich von pH 1,5 bis < 7 eingestellt und die R-Dihydroliponsäure oder S-Dihydroliponsäure oder einem Gemisch hiervon freigesetzt wird.

Bevorzugt wird das Verfahren derart durchgeführt, dass die erste Verfahrensstufe in einem alkoholischen Lösungsmittelgemisch bei einer Temperatur im Bereich von 100 °C bis 130 °C und bei einem Druck von 0,12 MPa bis 0,60 MPa, ganz besonders bevorzugt bei einer Temperatur im Bereich von 110 °C bis 130 °C und bei einem Druck von 0,25 MPa bis 0,50 MPa durchgeführt wird.

Gemäß einer bevorzugten Ausführung des Verfahrens kann insbesondere ein C1-bis C3-Alkylester der 6,8-Dichloroctansäure, insbesondere der racemischen 6,8-Dichloroctansäure, der R-Dichloroctansäure oder der S-Dichloroctansäure eingesetzt werden. Somit steht der Rest R in Formel (II), (IIa) oder (IIb) insbesondere für R = Methyl, Ethyl, oder n-Propyl. Diese Reste können besonders leicht unter vergleichsweise milden Reaktionsbedingungen zur Freisetzung des gewünschten Produktes abgespalten werden.

Bei dem erfindungsgemäß eingesetzten Natriumsulfid handelt es sich um Na₂S.

Bei dem erfindungsgemäß eingesetzten alkoholischen Lösungsmittelgemisch handelt es sich vorzugsweise um ein Gemisch aus einem oder mehreren Alkoholen und Wasser. Bevorzugt ist ein alkoholisches Lösungsgemisch umfassend einen oder mehrere Alkohole aus der Gruppe Methanol, Ethanol, Propanol und/oder iso-Propanol sowie Wasser. Es ist aber auch möglich als alkoholisches Lösungsmittelgemisch nur einen Alkohol oder ein Gemisch von zwei oder mehreren Alkoholen einzusetzen, insbesondere ein Gemisch umfassend zwei oder mehr Alkohole ausgewählt aus der Gruppe Methanol, Ethanol, Propanol und iso-Propanol.

Der Anteil an Alkohol im alkoholischen Lösungsmittelgemisch beträgt vorzugsweise 5 Gew.-% bis 100 Gew.-%, mehr bevorzugt mindestens 10 Gew.-% und noch mehr bevorzugt mindestens 15 Gew.-% und bis zu 80 Gew.-%, bevorzugt bis zu 60 Gew.-%, noch mehr bevorzugt bis zu 50 Gew.-%. Der Anteil an Wasser im alkoholischen Lösungsmittelgemisch beträgt bevorzugt 5 Gew.-% bis 95 Gew.-% und insbesondere mindestens 10 Gew.-%, mehr bevorzugt mindestens 20 Gew.-%, noch mehr bevorzugt mindestens 50 Gew.-% und bis zu 90 Gew.-% und insbesondere bis zu 80 Gew.-%.

Besonders bevorzugt ist das alkoholische Lösungsmittelgemisch ein Gemisch aus Ethanol und Wasser, am meisten bevorzugt ein Gemisch umfassend 20 Gew.-% bis 30 Gew.-% Ethanol und 70 Gew.-% bis 80 Gew.-% Wasser.

Gemäß der vorliegenden Erfindung wird das Verfahren derart durchgeführt, dass in der zweiten Verfahrensstufe das aus der ersten Verfahrensstufe erhaltene Reaktionsgemisch einer Reduktion unterworfen wird. Hierbei können eine Vielzahl an Reduktionsreaktionen eingesetzt werden. Im Rahmen einer weiter bevorzugten Ausführung der Erfindung ist vorgesehen, dass die Reduktion der zweiten Stufe durch Zugabe einer Lösung von Natriumborhydrid in einer Lauge erfolgt. Besonders bevorzugt kann hierbei als Lauge eine verdünnte oder konzentrierte Lauge, vorzugsweise eine verdünnte oder konzentrierte Natronlauge, Kalilauge oder eine Lösung umfassend Natriumcarbonat, Kaliumcarbonat verwendet werden. Gleichzeitig oder unabhängig hiervon kann die Reduktion in der zweiten Stufe besonders bevorzugt bei einer Temperatur im Bereich von 60 °C bis 85 °C und/oder bei Normaldruck durchgeführt werden.

Die somit eingestellten Reaktionsbedingungen für die Reduktion können als milde eingestuft werden und begünstigen den gewünschten Reaktionslauf besonders gut. Insbesondere werden hierdurch weiterhin weniger Nebenreaktionen beobachtet. Insbesondere wird auch die durch die erste Stufe besonders vorteilhaft zurückgedrängte Polymerisationsneigung der entstandenen Zwischenprodukte nicht begünstigt, so dass der Polymerisationsgrad der ersten Stufe beibehalten werden kann.

Gemäß einer weiter bevorzugten Ausführung des Verfahrens kann auch vorgesehen sein, dass in der ersten Stufe der Ester der 6,8-Dichloroctansäure und Natriumsulfid in einem molaren Verhältnis von Ester der 6,8-Dichloroctansäure zu Natriumsulfid im Bereich von 1 : 1 bis 1 : 2, insbesondere im Bereich von 1 : 1 bis 1 : 1,5, und weiter bevorzugt im Bereich von 1 : 1,1 bis 1 : 1,3, zur Reaktion gebracht werden. Insbesondere hat ein in Bezug auf den eingesetzten Ester der 6,8-Dichloroctansäure eingesetzter kleiner molarer Überschuss an Natriumsulfid zu guten Ergebnissen geführt.

Zudem kann weiterhin bevorzugt in der ersten Verfahrensstufe ein Gemisch aus Natriumsulfid und Schwefel eingesetzt werden, in dem das molare Verhältnis von Natriumsulfid zu Schwefel im Bereich von 0,8 : 1 bis 2 : 1, bevorzugt im Bereich von 0,8 : 1 bis 1,5 : 1, und besonders bevorzugt im Bereich von 0,8 : 1 bis 1,2 : 1, liegt.

In einer weiteren bevorzugten Ausführungsform wird in der ersten Verfahrensstufe ein Gemisch aus Natriumsulfid und Schwefel eingesetzt, in dem das molare Verhältnis von Natriumsulfid zu Schwefel im Bereich von 1 : 1 bis 2 : 1, bevorzugt im Bereich von 1 : 1 bis 1,6 : 1 und besonders bevorzugt im Bereich von 1,2 : 1 bis 1,5 : 1 liegt.

Gleichzeitig oder unabhängig hiervon kann dabei besonders bevorzugt vorgesehen sein, dass der Schwefel zusammen mit dem 6,8-Dichloroctansäureester, insbesondere dem racemischen 6,8-Dichloroctansäureester, dem R-Dichloroctansäureester oder dem S-Dichloroctansäureester in dem alkoholischen Lösungsmittelgemisch vorgelegt wird, und Natriumsulfid, insbesondere Natriumsulfid-Lösung, zugegeben wird. Weiterhin ist es bevorzugt, den Schwefel zusammen mit dem 6,8-Dichloroctansäureester, insbesondere einem racemischen 6,8-Dichloroctansäureester, einem R-Dichloroctansäureester oder einem S-Dichloroctansäureester in einem Alkohol oder Alkoholgemisch vorzulegen und dann Natriumsulfid, insbesondere eine wässrige Natriumsufid-Lösung zuzugeben.

In der dritten Verfahrensstufe in Schritt c) wird der pH-Wert erfindungsgemäß bevorzugt auf einen Bereich von pH 1,5 bis 5, mehr bevorzugt auf pH 1,5 bis 4 und am meisten bevorzugt auf pH 1,5 bis 3 eingestellt.

In einer besonderen Ausführungsform der Erfindung erfolgt die erfindungsgemäße Herstellung der Dihydroliponsäure in einem Eintopfverfahren, insbesondere ohne Isolierung oder Aufreinigung der Zwischenprodukte aus der ersten oder der zweiten Verfahrensstufe. Somit kann das Verfahren besonders effizient durchgeführt werden. Besonders überraschend ist hierbei, dass auch in Kenntnis der bekannten Reaktionswege von Schwefelverbindungen auch in der Reaktionsführung als Eintopfverfahren, keine nennenswerten Nebenprodukte gebildet werden. Diese Tatsache ist besonders überraschend und kann ohne an die Theorie gebunden zu sein, auf die in der ersten Verfahrensstufe eingestellten Reaktionsbedingungen zurückgeführt werden.

Somit ist auch ein Verfahren zur Herstellung von Dihydroliponsäure der hiermit beschriebenen Art Gegenstand der vorliegenden Erfindung, indem das Verfahren als Eintopf-Verfahren und/oder ohne Isolierung einer Zwischenstufe durchgeführt wird.

In diesem Verfahren kann, insbesondere in der ersten Verfahrensstufe, besonders bevorzugt als alkoholisches Lösungsmittelgemisch ein Alkohol ausgewählt aus der Gruppe Methanol, Ethanol, Propanol, iso-Propanol oder Mischungen hiervon oder Mischungen dieser Alkohole mit Wasser verwendet werden. Diese eingesetzten Alkohole können als absolute Lösungsmittel oder als technische Lösungsmittel mit einem Anteil an Wasser eingesetzt werden.

Letztendlich nicht einschränkend kann in der dritten Verfahrensstufe als Mineralsäure eine verdünnte oder konzentrierte Mineralsäure, vorzugsweise eine verdünnte oder konzentrierte Salzsäure, Salpetersäure oder Schwefelsäure, verwendet werden.

In einer weiteren vorteilhaften Ausgestaltung des erfindungsgemäßen Verfahrens wird in dem Schritt b), der im ersten Verfahrensschritt eingesetzte und im alkoholischen Lösungsgemisch enthaltene Alkohol zumindest teilweise, vorteilhafterweise nahezu vollständig entfernt. Insbesondere werden wenigstens 50 Gew.-%, mehr bevorzugt wenigstens 80 Gew.-%, noch mehr bevorzugt wenigstens 90 Gew.-% und am meisten bevorzugt wenigstens 95 Gew.-% und bis zu 100 Gew.-%, mehr bevorzugt bis zu 99 Gew.-% und noch mehr bevorzugt bis zu 98 Gew.-% des im ersten Verfahrensschritt eingesetzten Alkohols im Schritt b) entfernt. Vorteilhafterweise wird dabei zunächst ein erster Reduktionsschritt durchgeführt, dann der Alkohol entfernt und dann ein zweiter Reduktionsschritt ausgeführt.

Weiterhin bevorzugt wird erfindungsgemäß nach Schritt b) und vor Schritt c) ein Lösungsmittel für das gewünschte Produkt Dihydroliponsäure, insbesondere ein Ether und am meisten bevorzugt Methyl-tert-butylether (MTBE) zugegeben.

Die Zugabe dieses Lösungsmittels für das gewünschte Produkt Dihydroliponsäure und insbesondere die Zugabe eines Ethers erfolgt bevorzugt vor Zugabe einer Mineralsäure.
Zusammenfassend ist festzuhalten, dass die vorliegende Erfindung ermöglicht, Dihydroliponsäure auf wirtschaftliche Weise und in großtechnischem Maßstab zu gewinnen, die in bislang nicht bekannter Effizienz in alpha-Liponsäure oder physiologisch verträgliche Derivate überführt zu werden kann.

Die nachfolgenden Beispiele sollen die Vorteile des erfindungsgemäßen Verfahrens zur Herstellung von Dihydroliponsäure veranschaulichen.

### Beispiele

### Beispiel 1:

In einem 1000 ml Autoklav mit Rührer, Dosierpumpe, Druck- und Innentemperaturmessung sowie Mantelbeheizung werden 123,0 g (500 mmol) racemisches 6,8-Dichlorethylcaprylat und 14,4 g (450 mmol, 0,9 Äq.) Schwefel in 123,0 g Ethanol vorgelegt. Der Autoklav wird verschlossen und unter Rühren auf eine Innentemperatur von 110 °C aufgeheizt. Bei dieser Temperatur werden über einen Zeitraum von 60 min gleichmäßig 412,5 g einer wässrigen Natriumsulfid-Lösung (12,3 Gew.-% Na₂S in Wasser) umfassend 50,7 g Na₂S (650 mmol, 1,3 Äq.) zudosiert. Dabei steigt der Druck von anfangs 0,33 MPa auf 0,43 MPa an. Die Reaktionsmischung wird noch 120 min bei 110 °C gerührt, dann auf 50 °C abgekühlt und in einen 2000 ml Vierhalskolben mit Rührer, Dosierpumpe, Innenthermometer, Rückflusskühler und Ölbad-Heizung überführt. Bei 70 °C werden 94,6 g einer Lösung von Natriumborhydrid (12 Gew.-%) in Natronlauge (40 Gew.-%) umfassend 11,4 g NaBH₄ (300 mmol, 0,60 Äq.) innerhalb von 30 min gleichmäßig zugetropft. Der enthaltene Ethanol wird aus der Reaktionsmischung über einen Zeitraum von 60 min weitestgehend abdestilliert (bis Kopftemperatur 98 °C) und der Inhalt anschließend auf Raumtemperatur abgekühlt.

Die Reaktionsmischung wird am nächsten Tag bei 70 °C mit weiteren 47,3 g einer Lösung von Natriumborhydrid (12 Gew.-%) in Natronlauge (40 Gew.-%) umfassend 5,68 g NaBH₄ (150 mmol, 0,3 Äq.) über einen Zeitraum von 30 min versetzt. Nach erfolgter Zugabe wird die Mischung zum Sieden erhitzt und 30 min bei dieser Temperatur weiter gerührt. Nach dem Abkühlen werden 250 ml Methyl-tert-butylether (MTBE) zugegeben und der Ansatz durch zutropfen von 250,6 g (2200 mmol) Salzsäure (32 Gew.-%) auf einen pH-Wert von 1,5 bis 2,0 angesäuert. Die organische Phase wird in einem Scheidetrichter abgetrennt, die wässrige Phase mit 50 ml MTBE extrahiert und die vereinigten organischen Phasen über Magnesiumsulfat getrocknet. Es wird vom Trockenmittel abfiltriert und das Filtrat am Rotationsverdampfer vollständig eingeengt. Es werden 101,1 g einer gelben Flüssigkeit erhalten, mit einem Gehalt von 85,9 Gew.-% Dihydroliponsäure und einem Polymer-Gehalt von 11,4 Gew.-% (entspricht einer Ausbeute von 83,4 % d.Th.).

### Beispiel 2 (Vergleichsbeispiel nicht erfindungsgemäß):

In einem 2000 ml Vierhalskolben mit Rührer, Dosierpumpe, Innenthermometer und Rückflusskühler werden 123,0 g (500 mmol) racemisches 6,8-Dichlorethylcaprylat und 14,4 g (450 mmol, 0,9 Äq.) Schwefel in 123,0 g Ethanol vorgelegt und bei Normaldruck auf eine Innentemperatur von 75 °C erwärmt. Bei dieser Temperatur werden über einen Zeitraum von 60 min gleichmäßig 412,5 g einer wässrigen Natriumsulfid-Lösung (12,3 Gew.-% Na₂S in Wasser) umfassend 50,7 g Na₂S (650 mmol, 1,3 Äq.) zudosiert und die Reaktionsmischung anschließend noch weitere 120 min bei 75 °C gerührt. Der Reaktionsinhalt wird auf eine Temperatur von 70 °C abgekühlt und anschließend 94,6 g einer Lösung von Natriumborhydrid (12 Gew.-%) in Natronlauge (40 Gew.-%) umfassend 11,4 g NaBH₄ (300 mmol, 0,60 Äq.) innerhalb von 30 min gleichmäßig zugetropft. Der enthaltene Ethanol wird aus der Reaktionsmischung über einen Zeitraum von 60 min weitestgehend abdestilliert (bis Kopftemperatur 98 °C). Danach werden bei 70 °C weitere 47,3 g einer Lösung von Natriumborhydrid (12 Gew.-%) in Natronlauge (40 Gew.-%) umfassend 5,68 g NaBH₄ (150 mmol, 0,3 Äq.) über einen Zeitraum von 30 min zugetropft. Nach erfolgter Zugabe wird die Mischung zum Sieden erhitzt, für 30 min bei dieser Temperatur weiter gerührt und dann auf Raumtemperatur abgekühlt.

Zur Reaktionsmischung werden am nächsten Tag 250 ml MTBE zugegeben und der Ansatz durch Zutropfen von 250,9 g (2202 mmol) Salzsäure (32 Gew.-%) auf einen pH-Wert von 1,5 bis 2,0 angesäuert. Die organische Phase wird in einem Scheidetrichter abgetrennt, die wässrige Phase mit 50 ml MTBE extrahiert und die vereinigten organischen Phasen über Magnesiumsulfat getrocknet. Es wird vom Trockenmittel abfiltriert und das Filtrat am Rotationsverdampfer vollständig eingeengt. Es werden 99,9 g einer gelben Flüssigkeit erhalten, mit einem Gehalt von 75,6 Gew.-% Dihydroliponsäure und einem Polymer-Gehalt von 19,8 Gew.-% (entspricht einer Ausbeute von 72,5% d.Th.).

### Beispiel 3:

In einem 1000 ml Autoklav mit Rührer, Dosierpumpe, Druck- und Innentemperaturmessung sowie Mantelbeheizung werden 123,0 g (500 mmol) racemisches 6,8-Dichlorethylcaprylat und 16,0 g (500 mmol, 1,0 Äq.) Schwefel in 123,0 g Ethanol vorgelegt. Der Autoklav wird verschlossen und unter Rühren auf eine Innentemperatur von 110 °C aufgeheizt. Bei dieser Temperatur werden über einen Zeitraum von 60 min gleichmäßig 421,0 g einer wässrigen Natriumsulfid-Lösung (12,3 Gew.-%) umfassend 51,8 g Na₂S (663 mmol, 1,33 Äq) zudosiert. Dabei steigt der Druck von anfangs 0,29 MPa auf 0,45 MPa an. Die Reaktionsmischung wird noch 120 min bei 110 °C gerührt, dann auf 50 °C abgekühlt und in einen 2000 ml Vierhalskolben mit Rührer, Dosierpumpe, Innenthermometer, Rückflusskühler und Ölbad-Heizung überführt. Bei 70 °C werden 99,3 g einer Lösung von Natriumborhydrid (12 Gew.-%) in Natronlauge (40 Gew.-%) umfassend 11,9 g NaBH₄ (315 mmol, 0,63 Äq.) innerhalb von 30 min gleichmäßig zugetropft. Der enthaltene Ethanol wird aus der Reaktionsmischung über einen Zeitraum von 60 min weitestgehend abdestilliert (bis Kopftemperatur 98 °C) und der Inhalt anschließend auf Raumtemperatur abgekühlt.

Die Reaktionsmischung wird am nächsten Tag bei 70 °C mit weiteren 49,7 g einer Lösung von Natriumborhydrid (12 Gew.-%) in Natronlauge (40 Gew.-%) umfassend 5,96 g NaBH₄ (158 mmol, 0,32 Äq.) über einen Zeitraum von 30 min versetzt. Nach erfolgter Zugabe wird die Mischung zum Sieden erhitzt und 30 min bei dieser Temperatur weiter gerührt. Nach dem Abkühlen werden 250 ml MTBE zugegeben und der Ansatz durch Zutropfen von 263,4 g (2312 mmol) Salzsäure (32 Gew.-%) auf einen pH-Wert von 1,5 bis 2,0 angesäuert. Die organische Phase wird in einem Scheidetrichter abgetrennt, die wässrige Phase mit 50 ml MTBE extrahiert und die vereinigten organischen Phasen über Magnesiumsulfat getrocknet. Es wird vom Trockenmittel abfiltriert und das Filtrat am Rotationsverdampfer vollständig eingeengt. Es werden 102,3 g einer gelben Flüssigkeit erhalten, mit einem Gehalt von 89,8 Gew.-% Dihydroliponsäure und einem Polymer-Gehalt von 9,8 Gew.-% (entspricht einer Ausbeute von 88,2 % d.Th.).

### Beispiel 4:

In einem 6 m³ Reaktor mit Rührer, Dosierpumpe, Druck- und Innentemperaturmessung sowie Mantelbeheizung werden 600 kg (2,48 kmol) racemisches 6,8-Dichlorethylcaprylat und 78 kg (2,43 kmol, 0,98 Äq.) Schwefel in 576 kg Ethanol vorgelegt. Bei einer Innentemperatur von 110 °C und einem Druck von 0,35 MPa werden unter Rühren über einen Zeitraum von 85 min gleichmäßig 2062 kg einer wässrigen Natriumsulfid-Lösung (12,3 Gew.-%) umfassend 253,6 kg Na₂S (3,24 kmol, 1,31 Äq.) zudosiert. Die Reaktionsmischung wird noch 120 min bei 110 °C gerührt. Bei 70 °C werden anschließend 508 kg einer Lösung von Natriumborhydrid (12 Gew.-%) in Natronlauge (40 Gew.-%) umfassend 61,0 kg NaBH₄ (1,61 kmol, 0,65 Äq.) innerhalb von 90 min gleichmäßig zudosiert. Der enthaltene Ethanol wird aus der Reaktionsmischung weitestgehend abdestilliert und daraufhin bei 70 °C weitere 253 kg einer Lösung von Natriumborhydrid (12 Gew.-%) in Natronlauge (40 Gew.-%) umfassend 30,4 kg NaBH₄ (0,80 kmol, 0,32 Äq.) über einen Zeitraum von 60 min versetzt. Nach erfolgter Zugabe wird die Mischung zum Sieden erhitzt und 30 min bei dieser Temperatur weiter gerührt. Nach dem Abkühlen werden 540 kg MTBE zugegeben und der Ansatz durch Zugabe von 1454 kg (12,3 kmol) Salzsäure (31 Gew.-%) auf einen pH-Wert von 2,6 bis 3,0 angesäuert. Die organische Phase wird abgetrennt und das organische Lösungsmittel ohne Vakuum vollständig abdestilliert. Es werden 506 kg einer gelben Flüssigkeit erhalten, mit einem Gehalt von 94,0 Gew.-% Dihydroliponsäure und einem Polymer-Gehalt von 5,6 Gew.-% (entspricht einer Ausbeute von 91,8% d.Th.).

### Beispiel 5:

In einem 1000 ml Autoklav mit Rührer, Dosierpumpe, Druck- und Innentemperaturmessung sowie Mantelbeheizung werden 123,0 g (500 mmol) racemisches 6,8-Dichlorethylcaprylat und 14,4 g (450 mmol, 0,9 Äq.) Schwefel in 123,0 g Ethanol vorgelegt. Der Autoklav wird verschlossen und unter Rühren auf eine Innentemperatur von 100 °C aufgeheizt. Bei dieser Temperatur werden über einen Zeitraum von 60 min gleichmäßig 412,5 g einer wässrigen Natriumsulfid-Lösung (12,3 Gew.-% Na₂S in Wasser) umfassend 50,7 g Na₂S (650 mmol, 1,3 Äq.) zudosiert. Dabei steigt der Druck von anfangs 0,23 MPa auf 0,38 MPa an. Die Reaktionsmischung wird noch 120 min bei 100 °C gerührt, dann auf 50 °C abgekühlt und in einen 2000 ml Vierhalskolben mit Rührer, Dosierpumpe, Innenthermometer, Rückflusskühler und Ölbad-Heizung überführt. Bei 70 °C werden 94,6 g einer Lösung von Natriumborhydrid (12 Gew.-%) in Natronlauge (40 Gew.-%) umfassend 11,4 g NaBH₄ (300 mmol, 0,60 Äq.) innerhalb von 30 min gleichmäßig zugetropft. Der enthaltene Ethanol wird aus der Reaktionsmischung über einen Zeitraum von 60 min weitestgehend abdestilliert (bis Kopftemperatur 98 °C) und der Inhalt anschließend auf Raumtemperatur abgekühlt.

Die Reaktionsmischung wird am nächsten Tag bei 70 °C mit weiteren 47,3 g einer Lösung von Natriumborhydrid (12 Gew.-%) in Natronlauge (40 Gew.-%) umfassend 5,68 g NaBH₄ (150 mmol, 0,3 Äq.) über einen Zeitraum von 30 min versetzt. Nach erfolgter Zugabe wird die Mischung zum Sieden erhitzt und 30 min bei dieser Temperatur weiter gerührt. Nach dem Abkühlen werden 250 ml Methyl-tert-butylether (MTBE) zugegeben und der Ansatz durch zutropfen von 251,5 g (2207 mmol) Salzsäure (32 Gew.-%) auf einen pH-Wert von 1,5 bis 2,0 angesäuert. Die organische Phase wird in einem Scheidetrichter abgetrennt, die wässrige Phase mit 50 ml MTBE extrahiert und die vereinigten organischen Phasen über Magnesiumsulfat getrocknet. Es wird vom Trockenmittel abfiltriert und das Filtrat am Rotationsverdampfer vollständig eingeengt. Es werden 99,4 g einer gelben Flüssigkeit erhalten, mit einem Gehalt von 83,2 Gew.-% Dihydroliponsäure und einem Polymer-Gehalt von 12,0 Gew.-% (entspricht einer Ausbeute von 79,4% d.Th.).

### Beispiel 6:

In einem 1000 ml Autoklav mit Rührer, Dosierpumpe, Druck- und Innentemperaturmessung sowie Mantelbeheizung werden 123,0 g (500 mmol) racemisches 6,8-Dichlorethylcaprylat und 14,4 g (450 mmol, 0,9 Äq.) Schwefel in 123,0 g Ethanol vorgelegt. Der Autoklav wird verschlossen und unter Rühren auf eine Innentemperatur von 120 °C aufgeheizt. Bei dieser Temperatur werden über einen Zeitraum von 60 min gleichmäßig 412,5 g einer wässrigen Natriumsulfid-Lösung (12,3 Gew.-% Na₂S in Wasser) umfassend 50,7 g Na₂S (650 mmol, 1,3 Äq.) zudosiert. Dabei steigt der Druck von anfangs 0,35 MPa auf 0,46 MPa an. Die Reaktionsmischung wird noch 120 min bei 120 °C gerührt, dann auf 50 °C abgekühlt und in einen 2000 ml Vierhalskolben mit Rührer, Dosierpumpe, Innenthermometer, Rückflusskühler und Ölbad-Heizung überführt. Bei 70 °C werden 94,6 g einer Lösung von Natriumborhydrid (12 Gew.-%) in Natronlauge (40 Gew.-%) umfassend 11,4 g NaBH₄ (300 mmol, 0,60 Äq.) innerhalb von 30 min gleichmäßig zugetropft. Der enthaltene Ethanol wird aus der Reaktionsmischung über einen Zeitraum von 60 min weitestgehend abdestilliert (bis Kopftemperatur 98 °C) und der Inhalt anschließend auf Raumtemperatur abgekühlt.

Die Reaktionsmischung wird am nächsten Tag bei 70 °C mit weiteren 47,3 g einer Lösung von Natriumborhydrid (12 Gew.-%) in Natronlauge (40 Gew.-%) umfassend 5,68 g NaBH₄ (150 mmol, 0,3 Äq.) über einen Zeitraum von 30 min versetzt. Nach erfolgter Zugabe wird die Mischung zum Sieden erhitzt und 30 min bei dieser Temperatur weiter gerührt. Nach dem Abkühlen werden 250 ml Methyl-tert-butylether (MTBE) zugegeben und der Ansatz durch zutropfen von 251,3 g (2206 mmol) Salzsäure (32 Gew.-%) auf einen pH-Wert von 1,5 bis 2,0 angesäuert. Die organische Phase wird in einem Scheidetrichter abgetrennt, die wässrige Phase mit 50 ml MTBE extrahiert und die vereinigten organischen Phasen über Magnesiumsulfat getrocknet. Es wird vom Trockenmittel abfiltriert und das Filtrat am Rotationsverdampfer vollständig eingeengt. Es werden 100,6 g einer gelben Flüssigkeit erhalten, mit einem Gehalt von 87,5 Gew.-% Dihydroliponsäure und einem Polymer-Gehalt von 11,1 Gew.-% (entspricht einer Ausbeute von 84,5% d.Th.).

## Patentansprüche

1. Mehrstufiges Verfahren zur Herstellung von Dihydroliponsäure indem
a) in einer ersten Verfahrensstufe ein Ester der 6,8-Dichloroctansäure gemäß allgemeiner Formel (II) mit einem Gemisch umfassend Natriumsulfid und Schwefel zur Reaktion gebracht wird, wobei für Formel (II) gilt 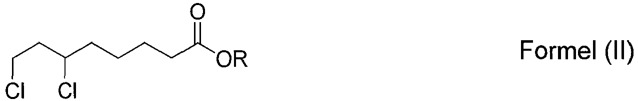 wobei für den Rest R gilt:
R = C1- bis C6-Alkyl,
wobei die Reaktion in einem alkoholischen Lösungsmittelgemisch bei einer Temperatur im Bereich von 80 °C bis 130 °C und einem Druck im Bereich von 0,12 MPa bis 0,60 MPa durchgeführt wird,
b) in einer zweiten Verfahrensstufe das aus der ersten Verfahrensstufe erhaltene Reaktionsgemisch einer Reduktion unterworfen wird und
c) in einer dritten Verfahrensstufe das aus der zweiten Verfahrensstufe resultierende Reaktionsgemisch mittels einer Mineralsäure auf einen pH-Wert im Bereich von pH 1,5 bis < 7 eingestellt und Dihydroliponsäure freigesetzt wird.

2. Verfahren nach Anspruch 1, **dadurch gekennzeichnet, dass** für den Rest R in Formel (II) gilt:
R = Methyl, Ethyl, oder n-Propyl.

3. Verfahren nach mindestens einem der vorhergehenden Ansprüche, **dadurch gekennzeichnet, dass** die Reduktion der zweiten Stufe durch Zugabe einer Lösung von Natriumborhydrid in einer Lauge erfolgt.

4. Verfahren nach Anspruch 3 **dadurch gekennzeichnet, dass** als Lauge eine verdünnte oder konzentrierte Lauge, vorzugsweise eine verdünnte oder konzentrierte Natronlauge, Kalilauge oder eine Lösung umfassend Natriumcarbonat, Kaliumcarbonat verwendet wird.

5. Verfahren nach mindestens einem der vorhergehenden Ansprüche, **dadurch gekennzeichnet, dass** die Reduktion in der zweiten Stufe bei einer Temperatur im Bereich von 60 °C bis 85 °C und/oder bei Normaldruck durchgeführt wird.

6. Verfahren nach mindestens einem der vorhergehenden Ansprüche, **dadurch gekennzeichnet, dass** das Verfahren als Eintopf-Verfahren und/oder ohne Isolierung einer Zwischenstufe durchgeführt wird.

7. Verfahren nach mindestens einem der vorhergehenden Ansprüche, **dadurch gekennzeichnet, dass** in der ersten Stufe der Ester der 6,8-Dichloroctansäure und Natriumsulfid in einem molaren Verhältnis von Ester der 6,8-Dichloroctansäure zu Natriumsulfid im Bereich von 1 : 1 bis 1 : 2 zur Reaktion gebracht wird.

8. Verfahren nach mindestens einem der vorhergehenden Ansprüche, **dadurch gekennzeichnet, dass** das Gemisch umfassend Natriumsulfid und Schwefel der ersten Verfahrensstufe Natriumsulfid und Schwefel in einem molaren Verhältnis von Natriumsulfid zu Schwefel im Bereich von 0,8 : 1 bis 2 : 1 enthält.

9. Verfahren nach mindestens einem der vorhergehenden Ansprüche, **dadurch gekennzeichnet, dass** als alkoholisches Lösungsmittelgemisch ein Alkohol ausgewählt aus der Gruppe Methanol, Ethanol, Propanol, iso-Propanol oder Mischungen hiervon oder Mischungen dieser Alkohole mit Wasser verwendet wird.

10. Verfahren nach mindestens einem der vorhergehenden Ansprüche **dadurch gekennzeichnet, dass** als Mineralsäure eine verdünnte oder konzentrierte Mineralsäure, vorzugsweise eine verdünnte oder konzentrierte Salzsäure, Salpetersäure oder Schwefelsäure verwendet wird.

## Claims

1. Multi-stage process for preparing the dihydrolipoic acid, in which
a) in a first process stage, an ester of 6,8-dichloroctanoic acid according to general formula (II) is reacted with a mixture comprising sodium sulfide and sulfur, wherein for formula (II) the following applies: 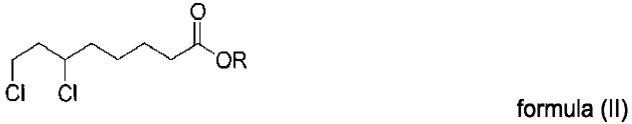 wherein for the group R the following applies:
R = C1 to C6 alkyl,
wherein the reaction is carried out in an alcoholic solvent mixture at a temperature in the range of from 80°C to 130°C and a pressure in the range of from 0.12 MPa to 0.60 MPa,
b) in a second process stage, the reaction mixture obtained from the first process stage is subjected to a reduction and
c) in a third process stage, the reaction mixture resulting from the second process stage is adjusted to a pH in the range of from pH 1.5 to < 7 by means of a mineral acid, and dihydrolipoic acid is released.

2. Process according to claim 1, **characterised in that** for the group R in formula (II) the following applies:
R = methyl, ethyl, or n-propyl.

3. Process according to at least one of the preceding claims, **characterised in that** the reduction of the second stage is carried out by adding a solution of sodium borohydride in a lye.

4. Process according to claim 3, **characterised in that** a diluted or concentrated lye, preferably a diluted or concentrated sodium hydroxide solution, potassium hydroxide solution, or a solution comprising sodium carbonate or potassium carbonate, is used as the lye.

5. Process according to at least one of the preceding claims, **characterised in that** the reduction in the second stage is carried out at a temperature in the range of from 60°C to 85°C and/or at normal pressure.

6. Process according to at least one of the preceding claims, **characterised in that** the process is carried out as a one-pot process and/or without isolation of an intermediate stage.

7. Process according to at least one of the preceding claims, **characterised in that**, in the first stage, the ester of 6,8-dichloroctanoic acid and sodium sulfide are reacted in a molar ratio of ester of 6,8-dichloroctanoic acid to sodium sulfide in the range of from 1:1 to 1:2.

8. Process according to at least one of the preceding claims, **characterised in that** the mixture comprising sodium sulfide and sulfur of the first process stage contains sodium sulfide and sulfur in a molar ratio of sodium sulfide to sulfur in the range of from 0.8:1 to 2:1.

9. Process according to at least one of the preceding claims, **characterised in that** an alcohol selected from the group consisting of methanol, ethanol, propanol, iso-propanol or mixtures thereof or mixtures of these alcohols with water is used as the alcoholic solvent mixture.

10. Process according to at least one of the preceding claims, **characterised in that** a diluted or concentrated mineral acid, preferably a diluted or concentrated hydrochloric acid, nitric acid or sulfuric acid, is used as the mineral acid.

## Revendications

1. Procédé en plusieurs étapes pour la préparation d'acide dihydroliponique où
a) dans une première étape de procédé un ester d'acide 6,8-dichloroctanoïque selon la formule générale (II) est mis à réagir avec un mélange comprenant du sulfure de sodium et du soufre, où pour la formule (II) s'applique 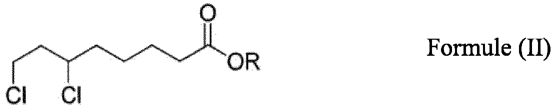 où pour le radical R s'applique:
R = C₁ à C₆-alkyle,
où la réaction est conduite dans un mélange de solvants alcooliques à une température dans le domaine de 80°C à 130°C et une pression dans le domaine de 0,12 MPa à 0,60 MPa,
b) dans une deuxième étape de procédé, le mélange réactionnel obtenu à partir de la première étape de procédé est soumis à une réduction et
c) dans une troisième étape de procédé, le mélange réactionnel résultant de la deuxième étape de procédé est ajusté à une valeur de pH dans le domaine de pH 1,5 à <7 à l'aide d'un acide minéral et l'acide dihydroliponique est libéré.

2. Procédé selon la revendication 1, **caractérisé en ce que** pour le radical R dans la formule (II) s'applique:
R = méthyle, éthyle ou n-propyle.

3. Procédé selon au moins l'une des revendications précédentes, **caractérisé en ce que** la réduction de la deuxième étape a lieu par addition d'une solution de borohydrure de sodium dans une lessive.

4. Procédé selon la revendication 3 **caractérisé en ce qu'**une lessive diluée ou concentrée, de préférence une lessive sodique, une lessive potassique ou une solution comprenant du carbonate de sodium, du carbonate de potassium diluée ou concentrée est utilisée comme lessive.

5. Procédé selon au moins l'une des revendications précédentes, **caractérisé en ce que** la réduction dans la deuxième étape est réalisée à une température dans le domaine de 60°C à 85°C et/ou à la pression normale.

6. Procédé selon au moins l'une des revendications précédentes, **caractérisé en ce que** le procédé est mis en oeuvre sous la forme d'un procédé monotope et/ou sans isolement d'une étape intermédiaire.

7. Procédé selon au moins l'une des revendications précédentes, **caractérisé en ce que** dans la première étape l'ester d'acide 6,8-dichloroctanoïque et le sulfure de sodium est mis à réagir dans un rapport molaire de l'ester d'acide 6,8-dichloroctanoïque au sulfure de sodium dans le domaine de 1:1 à 1:2.

8. Procédé selon au moins l'une des revendications précédentes, **caractérisé en ce que** le mélange comprenant du sulfure de sodium et du soufre de la première étape de procédé contient du sulfure de sodium et du soufre dans un rapport molaire du sulfure de sodium au soufre dans le domaine de 0,8:1 à 2:1.

9. Procédé selon au moins l'une des revendications précédentes, **caractérisé en ce qu'**un alcool choisi dans le groupe méthanol, éthanol, propanol, isopropanol ou des mélanges de ceux-ci ou des mélanges de ces alcools avec de l'eau est utilisé comme mélange de solvants alcooliques.

10. Procédé selon au moins l'une des revendications précédentes, **caractérisé en ce qu'**un acide minéral dilué ou concentré, de préférence un acide chlorhydrique, un acide nitrique ou un acide sulfurique dilué ou concentré est utilisé comme acide minéral.
